**Europäisches Patentamt**

**European Patent Office**

**Office européen des brevets**

(11) Veröffentlichungsnummer: **0 205 974 B1**

(19)

(12) # EUROPÄISCHE PATENTSCHRIFT

(45) Veröffentlichungstag der Patentschrift: **15.01.92**

(21) Anmeldenummer: **86107275.9**

(22) Anmeldetag: **28.05.86**

(51) Int. Cl.⁵: **A61L 15/16**, A61M 35/00

(54) **Arzneipflaster für systemische Anwendungen.**

(30) Priorität: **20.06.85 DE 3522060**

(43) Veröffentlichungstag der Anmeldung:
**30.12.86 Patentblatt 86/52**

(45) Bekanntmachung des Hinweises auf die
Patenterteilung:
**15.01.92 Patentblatt 92/03**

(84) Benannte Vertragsstaaten:
**AT BE CH DE FR GB IT LI NL SE**

(56) Entgegenhaltungen:
**EP-A- 0 153 200**
**EP-A- 0 164 726**
**EP-A- 0 174 108**
**DE-A- 3 503 111**
**US-A- 4 439 194**

**MANUFACTURING CHEMIST, Band 56, Nr. 5,
Mai 1985, Seite 47, London, GB; "A UK first in
transdermal dosage"**

(73) Patentinhaber: **Liedtke, Rainer K., Dr.
Tölzer Strasse 36
W-8022 Grünwald(DE)**

(72) Erfinder: **Liedtke, Rainer K., Dr.
Tölzer Strasse 36
W-8022 Grünwald(DE)**

(74) Vertreter: **Bühling, Gerhard, Dipl.-Chem. et al
Patentanwaltsbüro Tiedtke-Bühling-Kinne
Grupe-Pellmann-Grams-Struif Bavariaring 4
W-8000 München 2(DE)**

**Beschreibung**

Die Erfindung betrifft ein Arzneipflaster, insbesondere zur Verbesserung der Hautabsorption von dermal applizierten Arzneistoffen.

Es ist bekannt, daß mit Arzneipflastern oder sogenannten therapeutischen Pflastersystemen, neuerdings auch als transdermale therapeutische Systeme bezeichnet, auch systemische Arzneimittelwirkungen erzielt werden können. Praktische Anwendung mit dieser Applikationsform finden hierbei bereits die Wirkstoffe Scopolamin bei Kinetosen, Nitroglycerin bei Koronarer Herzerkrankung und Clonidin bei Hypertonie sowie transdermal verabreichte Östrogene. Die bisher eingesetzten Pflastersysteme sind jedoch technisch komplex und die erzielbaren Absorptionsraten, gemessen über die systemischen Arzneistoffkonzentrationen im Blut, liegen deutlich unter denen nach oralen Verabreichungen. Die Systeme zeigen zudem bezüglich der ermittelten Serumkonzentrationen der verabreichten Arzneistoffe erhebliche interindividuelle Schwankungen.

Die Pflastersysteme sind als Diffusionseinheiten konzipiert, bei denen die Arzneistoffe aus einem mechanisch festen Arzneimittelreservoir, in der Regel gewebeverträglichen Polymeren, durch Diffusion in kontrollierten Raten freigesetzt werden. Die eingesetzten Systeme werden dabei derzeit unterteilt in Membransysteme (Membranpflaster) und Matrixsysteme. Bei den Membransystemen müssen die Arzneistoffe nach der Freisetzung aus der Trägermasse noch eine Membran, die als Kontrollelement für eine gleichförmige Absorptionsrate dient, durchdringen um eine Freisetzungscharakteristik, die annähernd einer Pharmakokinetik Nullter Ordnung entspricht, zu erzielen. Bei den Matrixsystemen diffundiert der in Depotform gespeicherte Arzneistoff direkt aus der Polymermatrix in die Haut.

Da das Übertreten der Wirkstoffe aus dem Pflastersystem in die Haut nach den Diffusionsgesetzen erfolgt, die in den Fick'schen Diffusionsprinzipien quantifiziert sind:

$$\frac{dQ}{dt} = D \cdot F \frac{c_1 - c_2}{d}$$

wobei pro Zeiteinheit (t) die transportierte Wirkstoffmenge (Q)( = Diffusionsrate) abhängig ist von dem Diffusionskoeffizienten (D), der Austauschfläche (F) und der Konzentrationsdifferenz ($c_1 - c_2$) sowie der Diffusionsstrecke bzw. der Schichtdicke (d), läßt sich feststellen, daß Pflaster mit mechanisch starrer Matrix den Diffusionsbedingungen nicht optimal folgen.

So legen sich Polymermatrices oder technische Membranen der biologisch individuell und unregelmäßig geformten komplementären Hautoberfläche, die die Austauschfläche darstellt, annähernd plan an. Dies bedeutet eine unvollständige Nutzung der biologisch verfügbaren Absorptionsfläche. Gleichzeitig wird die Diffusionsstrecke hierdurch in einigen Bereichen der aufliegenden Absorptionsfläche des Pflasters vergrößert. Beide Effekte bedeuten eine Verschlechterung der allgemeinen Diffusionsbedingungen. Da die Geschwindigkeit des Diffusionsprozesses darüber hinaus auch von der Temperatur abhängig ist, wird, bei der unvollständigen Auflagerung, der Temperaturaustausch zwischen technischem Resorptionsareal und Arzneireservoir einerseits und Hautoberfläche andererseits, nicht optimal erreicht. Ein weiterer nachteilhafter Effekt ist die nur relativ langsame Wasseraufnahme, die für den Lösungsvorgang der Arzneistoffe erforderlich ist. Die Herstellung der Membran- und Matrixsysteme ist zudem technisch aufwendig und erfordert spezielle Apparaturen, wodurch höhere Kosten als bei der Herstellung oraler Anwendungsformen verursacht werden.

Aus der Literaturstelle "Manufacturing Chemist", May 1985, Seite 47, ist ein Arzneimittelpflaster bekannt, bei dem die Arzneistoffe in einer als Gel oder Creme ausgebildeten Trägermasse in einem auf der Haut fixierbaren Gehäuse enthalten sind. Die Cremes sind Wasser in Öl bzw. Öl in Wasseremulsionen und zeigen ebenso wie Gele kein Fest/Flüssig-Schmelzverhalten. Die Wirkstoff-Freisetzung erfolgt bei Cremes und Gelen ausschließlich aus der vorhandenen wässrigen Phase durch Diffusion. Die bekannte Trägermasse enthält darüber hinaus Enhancer bzw. Penetrationsbeschleuniger, wie Dimethylsulfoxid, Natriumlaurylsulfonat und Mischungen von Propylenglykol oder Ölsäure. Diese Enhancer dienen dazu, den Durchgang der Arzneimittel durch die Haut zu unterstützen, die normalerweise nicht durch die Haut eindringen können. Die erwähnten Enhancer greifen chemisch in die Integrität der biologischen Hautbarriere ein und führen daher zwangsläufig zu Integrationsstörungen der oberen Hautschichten. Hierdurch ergeben sich grundsätzlich Probleme bezüglich der Hautsicherheit.

Der Erfindung liegt die Aufgabe zugrunde, die Hautabsorption von dermal applizierten Arzneimitteln zu verbessern.

Diese Aufgabe wird gelöst durch ein Arzneipflaster zur Verbesserung der Hauptabsorption dermal applizierter Arzneistoffe mit einem auf der Haut fixierbaren Gehäuse, das Arzneistoffe in einer als Scheibe ausgebildeten Trägermasse enthält, wobei das Gehäuse nach oben abgeschlossen und zur Hautoberfläche offen ist, das dadurch gekennzeichnet ist, daß die Scheibe aus einer festen Trägermasse aus Hartfetten/Adeps solidus, Mischungen aus verschiedenen Hartfetten oder Mischungen aus Gelatine mit Hartfetten gebildet ist, wobei die feste Trägermasse bei Körpertemperatur schmilzt, so daß das Arzneimittel aus der flüssigen Phase freigegeben wird, daß die Trägermassenscheibe an der Unterseite einer porösen und elastischen Kunststoffscheibe aus Polyurethan-Schaumstoff vom Äther-Typ oder Ester-Typ gleichen Durchmessers fixiert ist, indem die Trägermasse mit der Kunststoffscheibe durch wenigstens partielles Eindringen der Trägermasse in die Poren der Kunststoffscheibe mechanisch verbunden ist und daß die beiden miteinander verbundenen Teile sich in dem Gehäuse befinden.

Um gleichzeitig auch eine verzögerte Wirkstoffreisetzung zu ermöglichen, wird, in einer weiteren Ausbildung der Erfindung, der Wirkstoff nicht homogen gelöst, sondern in einer pharmazeutisch-technischen Depotform in der Trägermasse aufge-nommen.

Um unterschiedliche successive Absorptionsra-ten zu erzielen, werden, in einer weiteren Ausbildung der Erfindung, zwei oder mehrere Schichten an Trägermassenscheiben mit jeweils unterschied-lichem Schmelzverhalten übereinander aufgetra-gen.

Um die Gesamtelastizität der Kunststoffscheibe besser zu erhalten, wird, in einer weiteren Ausbildung der Erfindung, die Trägermasse in den Poren der gesamten Kunststoffscheibe verteilt.

Um eine bessere mechanische Trennung zwischen Trägermasse und Gehäuse zu erhalten, wird, in einer weiteren Ausbildung der Erfindung, die Kunststoffscheibe auf der Oberseite mit einer mechanischen Trennschicht versehen.

Um besonders günstige physikalische Eigenschaften, verbunden mit einer physiologischen Indifferenz, zu erzielen, wird, die Kunststoffscheibe aus Polyurethan-Schaumstoff vom Äther-Typ oder vom Ester-Typ ausgebildet.

Um besonders günstige physikalische und bio-pharmazeutische Eigenschaften, verbunden mit ei-ner guten physiologischen Verträglichkeit zu erzielen, werden Trägermassen aus Hartfetten/Adeps solidus, Mischungen aus verschiedenen Hartfetten oder Mischungen aus Gelatine Mit Hartfetten in die Poren einer Kunststoffscheibe aus Polyurethan-Schaumstoff vom Äther-Typ oder Ester-Typ eingebracht.

Um die Freisetzung lipophiler Wirkstoffe zu verbessern sowie eine Regulation der physikalischen und biopharmazeutischen Eigenschaften der Trägermasse zu bewirken, werden, in einer weiteren Ausbildung der Erfindung, in die Trägermasse mittelkettige Partialglyceride oder Mischungen aus Partialglyceriden eingebracht.

Um die Freisetzung hydrophiler Wirkstoffe aus der Trägermasse zu verbessern, werden, in einer weiteren Ausbildung der Erfindung, in die Trägermasse hydrophile Hilfsstoffe eingebracht.

Um eine verbesserte Hauthaftung des Arzneipflasters in Verbindung mit einem ausreichenden Okklusiveffekt, sowie einen besseren Schutz der Trägermassenscheibe vor thermischen und mechanischen Einflüssen zu erreichen, wird, in einer weiteren Ausbildung der Erfindung, eine einseitig selbstklebende Kunststoffolie in Verbindung mit einem einseitig selbstklebenden, geschlossenporigen Schaumstoffring eingesetzt, wobei die Trägermassenscheibe mit der Unterfläche der Kunststoffolie verbunden ist und sich in der Aussparung des Schaumstoffringes befindet.

Die mit der Erfindung erzielten Vorteile ergeben sich insbesondere dadurch, daß die Wirkstoffreisetzung aus der Unterseite der Trägermassenscheibe durch den von der Hauttemperatur induzierten Schmelzvorgang beschleunigt wird und der Übertritt in die Haut aus einer flüssigen Phase der Trägermasse erfolgt. Da sich die Trägermasse als ein flüssiger Film ausbreitet, wird die gesamte zur Verfügung stehende komplementäre Hautoberfläche auch in der Mikrotopographie bedeckt und, im Gegensatz zu den plan und damit unvollständig aufliegenden mechanisch inflexibleren Systemen, hierdurch auch ein Einbezug der tiefer liegenden Hautanhangsgebilde wie Talgdrüsen und Schweißdrüsen, die ein beträchtliches Absorptionsareal darstellen, erreicht. Durch die direkte Auflagerung der flüssigen Phase der Trägermasse entfällt die technische Notwendigkeit einer zusätzlichen Hartfolie im Absorptionsareal, wie dies bei mechanisch festen Systemen der Fall ist. Durch den innigen Kontakt der flüssigen Phase der Trägermasse mit der Haut bis in den mikrotopographischen Bereich wird gleichzeitig die durchschnittliche Diffusionsstrecke verringert. Die optimale Flächenausnutzung des zur Verfügung stehenden Hautabsorptionsareals und die Reduzierung der Diffusionsstrecke ergeben somit gegenüber mechanisch festen Systemen Vorteile bei den Diffusionsbedingungen. Die für die verschiedenen Arzneistoffe spezifischen Bedingungen des Transportes durch die Hautoberfläche, die nach den Gesetzmäßigkeiten der sogenannten 'non-ionic-diffusion' erfolgen, werden insgesamt begünstigt.

Die mit der successiv schmelzenden Trägermassenscheibe erzielten Effekte sind mit der äußerlichen Anwendung flüssiger oder visköser Zubereitungen wie Salben und Sprays, oder der innerlichen Anwendung von Magen-Gelen oder Suppositorien, vergleichbar. Im Gegensatz zu Salben und Sprays, kommt es aber nicht zu einer Austrocknung der Trägermasse durch Verdunstung und da-mit einer Verschlechterung der Löslichkeitsverhält-

nisse. Vielmehr wird bei dem Pflastersystem durch die Abdeckung eine feuchte Kammer erzeugt, die Ihrerseits die Penetration der Arzneistoffe, durch Anhebung der Hydratation des wasserarmen Stratum corneum, verbessert.

Da für jeden Wirkstoff, entsprechend dessen physikalisch-chemischen Eigenschaften spezifische, galenisch optimale Trägermassenscheiben hergestellt werden können, ist das System, bei gleicher Grundkonfiguration, vielseitig und technologisch einfach. Im Gegensatz zu den dermalen Anwendungen Salbe, Gel und Spray, ist das System exakt dosierbar. Es besteht zudem keine Gefahr der Verschmutzung oder Entfernung des Wirkstoffes durch äußerliche Einflüsse. Da zwischen Trägermasse und Haut keine mechanischen Komponenten wie Membran oder Haftfolien zwischengeschaltet sind, kommt es von dieser Seite auch zu keinen mechanischen Irritationen durch Reibungskräfte. Während Muskelbewegungen oder temporäre Flächenveränderungen des Klebeareals, z. B. bedingt durch Atemexkursionen im Thoraxbereich, bei mechanischen Systemen sowohl die Haftfähigkeit der Haftfolie beeinträchtigen können als auch einen ständigen mechanischen Reiz bewirken können, ist bei der Trägermassenscheibe, deren Unterseite stets als flüssige Phase vorliegt, dieser Effekt eher vorteilhaft, da dies die Verteilung und somit Oberflächenvergrößerung in der Mikrotopographie der Haut, ähnlich einer Salbeneinreibung, begünstigt.

Da die Herstellung der Trägerscheiben, z. B. durch einfache Gieß- oder Preßtechniken, wie bei der Herstellung von Suppositorien, weniger aufwendig ist als die Herstellung exakt dosierter Polymer-Matrices oder Membransysteme, können auch die Herstellungskosten verringert werden. In die Trägerscheibe können neben homogen verteilten Wirkstoffen auch pharmazeutisch-technisch retardierte Formulierungen aufgenommen werden, die eine eigenständige Freisetzungscharakteristik besitzen, so daß in dem System gleichzeitig eine schnelle als auch eine verlangsamte Absorptionskomponente realisiert wird. Eine weitere Möglichkeit der Steuerung unterschiedlicher Freisetzungscharakteristiken ist in der Auftragung von mehreren Trägermassenscheiben mit unterschiedlichem Schmelzverhalten gegeben. Durch das partielle Eindringen der Trägermasse in die Poren des elastischen Elementes, ist eine feste Verbindung zwischen den beiden Bestandteilen gewährleistet, so daß auch bei eventuellen Beschädigungen der Trägermassenscheibe im Festzustand, diese sich nicht, weder im Ganzen noch in Einzelteilen, vom elastischen Element löst. Das elastische Element gewährleistet zudem, unabhängig von der jeweiligen Lage der Pflasteranbringung, einen stetigen Andruck und somit eine feste Anlagerung der Trägermassenscheibe an die Hautoberfläche.

Weitere Effekte lassen sich über die Hauttemperatur, die die Geschwindigkeit des Schmelzvorganges steuert, erzielen. So wird bei erhöht vorliegender Hauttemperatur, z. B. bei fiebrigen Zuständen, der Schmelzvorgang und hieraus resultierend die Wirkstoffreisetzung beschleunigt. Bei nachlassender Hauttemperatur, die z. B. durch einen transdermal freigesetzten Wirkstoff mit antipyretischer Eigenschaft systemisch erzeugt sein kann, wird auch die Freisetzungsgeschwindigkeit wieder reduziert. Ein derartiger Vorgang entspricht einer direkten biologischen Rückkoppelung mit Gegensteuerungseffekt auf die Arzneimittelfreisetzung.

Ausführungsbeispiele der Erfindung sind in den Abbildungen dargestellt, wobei die Beispiele die Erfindung erläutern, ohne sie zu beschränken: Abbildung 1 zeigt das System im Querschnitt. Abbildung 2 zeigt das System als Explosionsschema. Abbildung 3 zeigt eine Ausschnittvergrößerung des Arzneipflasters im Querschnitt.

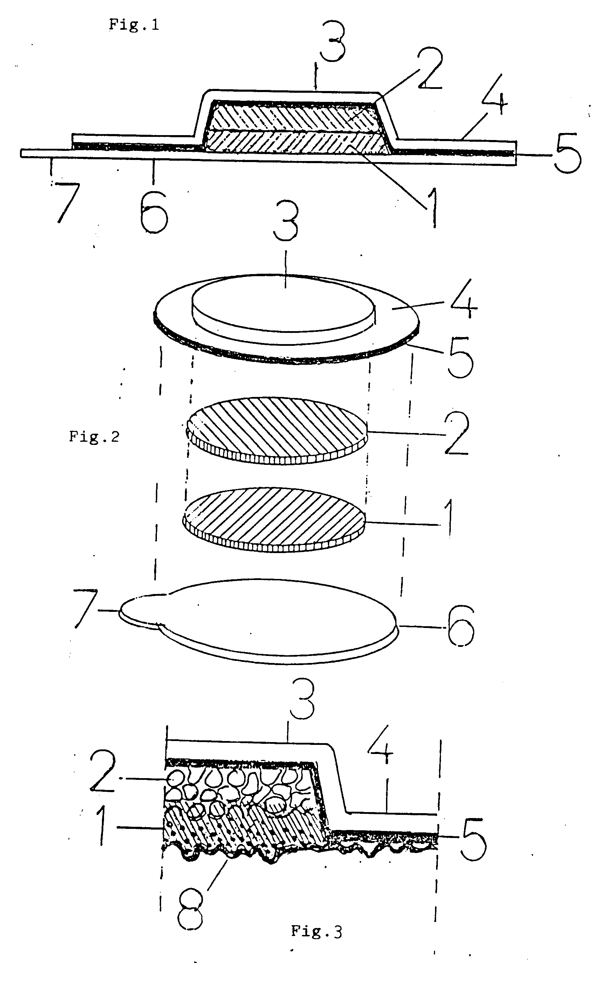

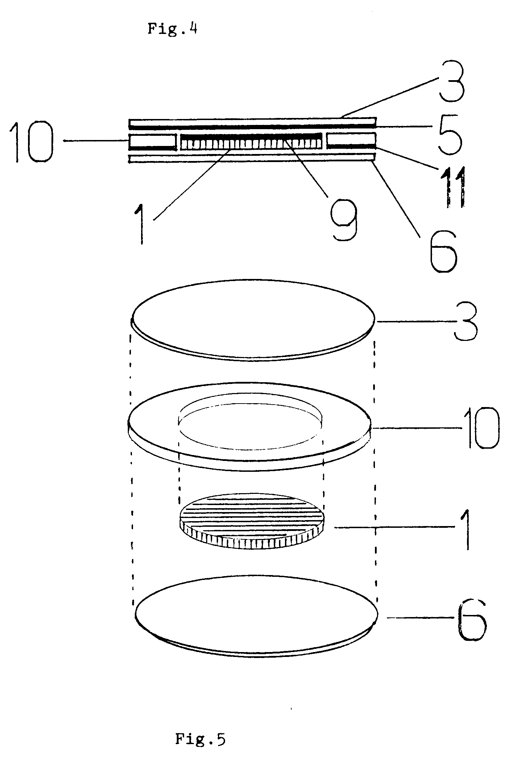

Der Arzneistoff befindet sich in der als flache Scheibe ausgebildeten Trägermasse (1). Diese ist ihrerseits an der Unterseite einer porösen und elastischen Kunststoffscheibe (2) befestigt, wobei Teile der Trägermasse in die Poren der Kunststoffscheibe eingelagert sind. Letzterer Effekt ist speziell in der Ausschnittvergrößerung des Querschnittes in Abb. 3 dargestellt. Trägermassenscheibe und Kunststoffscheibe befinden sich in einem als Abdeckschicht dienenden, nach oben geschlossenen Gehäuse (3), welches einen zirkulär verlaufenden Rand (4) besitzt. Sowohl die Innenseite des Gehäuses als auch die Unterseite des Randes ist mit einer Klebeschicht (5) versehen. Die Klebeschicht im Gehäuse dient der Fixation der Kunststoffscheibe, die Klebeschicht des Randes dient der Fixation des Pflasters auf der Hautoberfläche. Die offene Unterseite des Gehäuses mit der Trägermassenscheibe wird durch eine abziehbare Folie (6), die auf dem Rand klebt, fest verschlossen. Die Folie ist so dimensioniert, daß sie die gesamte Unterfläche einschließlich des Kleberandes abdeckt. An einer Seite ragt ein Teilstück der Folie über (7) den Kleberand. Dieses Teil ermöglicht ein leichteres Abziehen der Folie von der Klebefläche. In Abb. 3 ist neben der mechanischen Verbindung zwischen Trägermassenscheibe und Kunststoffscheibe durch Penetration der Trägermasse in die Poren der Kunststoffscheibe gleichzeitig auch schematisch die Verteilung der flüssigen Phase der Trägermasse auf der unregelmäßig strukturierten Mikrotopographie der Hautoberfläche (8) dargestellt.

In den Abbildungen 4 und 5 ist das System mit Detailmodifikationen dargestellt. Abbildung 4 zeigt eine Darstellung im Querschnitt, Abbildung 5 zeigt die Modifikation als Explosionsschema.

Bei der in den Abbildungen 4 und 5 dargestellten Modifikation des Grundsystemes ist die Gehäuseoberseite plan. Die obere Gehäuseseite besteht aus einer okklusiven Kunststofffolie (3), die auf der Unterseite mit einer Klebeschicht (5) versehen ist. Im Mittelteil der Unterseite der okklusiven Kunststofffolie ist die Oberseite der Kunststoffscheibe (1) angeklebt, wobei dieser Oberteil der Kunststoffscheibe als eine mechanische Trennschicht (9) ausgebildet ist, die eine Diffusion der Wirkstoffe in die Klebezone der okklusiven Kunststofffolie unterbindet. An das äußere Areal der Unterseite der Kunststofffolie ist zirkulär ein aus geschlossenporigem Polymermaterial bestehender Schaumstoffring (10) angeklebt, der seinerseits auf der Unterseite über eine Klebeschicht (11) verfügt. Diese Klebeschicht dient der Fixation des Arzneipflasters auf der Haut. Die Kunststoffscheibe mit der in ihr enthaltenen Trägermasse (1) befindet sich in der zentralen Aussparung des Schaumstoffringes (10). Auf der zur Hautfläche hingewandten Unterseite der Kunststoffscheibe, befindet sich eine mechanisch dichte, in den Dimensionen des Gesamtdurchmessers des Pflasters gehaltene Schutzfolie (6), die zirkulär mit dem Rand der Klebeschicht des Schaumstoffringes (11) verbunden ist und vor der Auflage auf die Haut entfernt werden kann.

In Abbildung 5 ist das Arzneipflaster als Explosionsschema dargestellt und zeigt die Hauptschichten Kunststofffolie (3), Schaumstoffring (10), Kunststoffscheibe mit Trägermasse (1) und die abziehbare Schutzfolie (6).

Als Arzneistoffe kommen zum Beispiel solche für die Behandlung von Bluthochdruck und Angina pectoris in Frage, wie Calcium-Antagonisten oder $\beta$-Blocker. Insbesondere kommen solche Arzneimittel in Frage, die aufgrund ihrer Physikochemischen-Eigenschaftenüber die Haut aufnehmbar sind.

**Patentansprüche**

1. Arzneipflaster zur Verbesserung der Hautabsorption dermal applizierter Arzneistoffe mit einem auf der Haut fixierbaren Gehäuse, das Arzneistoffe in einer als Scheibe ausgebildeten Trägermasse enthält, wobei das Gehäuse nach oben abgeschlossen und zur Hautoberfläche offen ist, **dadurch gekennzeichnet, daß die** Scheibe aus einer festen Trägermasse aus Hartfetten/Adeps solidus, Mischungen aus verschiedenen Hartfetten oder Mischungen aus Gelatine mit Hartfetten gebildet ist, wobei die feste Trägermasse bei Körpertemperatur schmilzt, so daß das Arzneimittel aus der flüssigen Phase freigegeben wird, daß die Trägermassenscheibe an der Unterseite einer porösen und elastischen Kunststoffscheibe aus Polyurethan-Schaumstoff vom Äther-Typ oder Ester-Typ gleichen Durchmessers fixiert ist, indem die Trägermasse mit der Kunststoffscheibe durch wenigstens partielles Eindringen der Trägermasse in die Poren der Kunststoffscheibe mechanisch verbunden ist und daß die beiden miteinander verbundenen Teile sich in dem Gehäuse befinden.

2. Arzneipflaster nach Anspruch 1, **dadurch gekennzeichnet, daß** die Wirkstoffe nicht ausschließlich homogen gelöst sind, sondern auch in einer technischen Depotform in der Trägermasse aufgenommen sind.

3. Arzneipflaster nach Anspruch 1 oder 2, **dadurch gekennzeichnet, daß** mehrere Trägermassenscheiben mit unterschiedlichem Schmelzverhalten übereinander aufgetragen sind.

4. Arzneipflaster nach wenigstens einem der vorhergehenden Ansprüche, **dadurch gekennzeichnet, daß** die Trägermasse in den Poren der gesamten Kunststoffscheibe verteilt ist.

5. Arzneipflaster nach wenigstens einem der vorhergehenden Ansprüche, **dadurch gekennzeichnet, daß** die Kunststoffscheibe auf der Oberseite mit einer mechanischen Trennschicht versehen ist.

6. Arzneipflaster nach wenigstens einem der vorhergehenden Ansprüche, **dadurch gekennzeichnet, daß** in die Trägermasse mittelkettige Partialglyceride oder Mischungen aus Partialglyceriden eingebracht sind.

7. Arzneipflaster nach wenigstens einem der vorhergehenden Ansprüche, **dadurch gekennzeichnet, daß** das Pflaster aus einer einseitig selbstklebenden Kunstoffolie besteht, an deren Unterseite sich ein einseitig selbstklebender, geschlossenporiger Schaumstoffring befindet und die mit der Kunststoffolie verbundene Trägermassenscheibe sich in der Aussparung des Schaumstoffringes befindet.

8. Arzneipflaster nach wenigstens einem der vorhergehenden Ansprüche, **dadurch gekennzeichnet, daß** als Arzneistoffe beispielsweise solche für die Behandlung von Bluthochdruck und Angina pectoris verwendbar sind.

**Claims**

1. A medicinal plaster for improving the skin absorption of dermally applied medicinal substances with a casing securable on the skin, which contains medicinal substances in a disc shaped carrier mass, the casing being closed towards the top and open towards the skin surface, characterized in that the disc is formed by a solid carrier mass of hard fats/adeps solidus, mixtures of various hard fats or mixtures of gelatine with hard fats, the solid carrier mass melting at body temperature so that the medicinal substance is released from the liquid phase, in that the carrier mass is fixed to the bottom of a porous and elastic synthetic disc made of an ether-type or ester-type polyurethane foam having the same diameter, the carrier mass being mechanically joined to the synthetic disc by at least a partial penetration of the carrier mass into the pores of the synthetic disc and in that the two parts joined to each other are located in the casing.

2. A medicinal plaster according to claim 1, characterized in that the active substances are not only dissolved homogeneously but are also absorbed in a technical deposit form in the carrier mass.

3. A medicinal plaster according to claim 1 or 2, characterized in that several discs of carrier mass with different melting properties are deposited on top of one another.

4. A medicinal plaster according to at least one of the preceding claims, characterized in that the carrier mass is distributed in the pores of the synthetic disc as a whole.

5. A medicinal plaster according to at least one of the preceding claims, characterized in that the synthetic disc is provided at its top with a mechanical separating layer.

6. A medicinal plaster according to at least one of the preceding claims, characterized in that medium chain partial glycerides or mixtures of partial glycerides are introduced into the carrier mass.

7. A medicinal plaster according to at least one of the preceding claims, characterized in that the plaster consists of a synthetic film that is self-adhesive on one side, at the bottom whereof there is disposed a foam ring with sealed pores which is self-adhesive on one side, and in that the carrier mass disc joined to the synthetic film is located in the pocket of the foam ring.

8. A medicinal plaster according to at least one of the preceding claims, characterized in that as medicinal substances, one may, for example, use those for the treatment of high blood pressure and angina pectoris.

**Revendications**

1. Pansement médicamenteux destiné à améliorer l'absorption à travers la peau de médicaments appliqués dermiquement, comportant un boîtier à fixer sur la peau qui contient des médicaments dans une masse de support en forme de disque, le boîtier étant fermé vers le haut et ouvert vers la surface de la peau, caractérisé en ce que le disque est formé d'une masse de support solide faite de graisses solides/Adeps solidus, de mélanges de différentes graisses solides ou de mélanges de gélatine avec des graisses solides, la masse de support solide fondant à la température corporelle ce qui fait que le médicament est libéré de la phase liquide, en ce que le disque de masse de support est fixé sur la face inférieure d'un disque en matière plastique poreux et élastique en mousse de polyuréthanne de type éther ou ester de même diamètre, la masse de support étant assemblée mécaniquement avec le disque en matière plastique par pénétration au moins partielle de la masse de support dans les pores du disque en matière plastique et en ce que les deux parties assemblées entre elles se trouvent dans le boîtier.

2. Pansement médicamenteux selon la revendication 1, caractérisé en ce que les substances actives ne sont pas dissoutes de manière exclusivement homogène mais sont absorbées aussi dans la masse de support sous la forme d'un dépôt technique.

3. Pansement médicamenteux selon la revendication 1 ou 2, caractérisé en ce que plusieurs disques de masse de support présentant un comportement à la fusion différent, sont appliqués de manière superposée.

4. Pansement médicamenteux selon l'une au moins des revendications précédentes, caractérisé en ce que la masse de support est répartie dans les pores de l'ensemble du disque en matière plastique.

5. Pansement médicamenteux selon l'une au moins des revendications précédentes, caractérisé en ce que le disque en matière plastique est pourvu sur sa face supérieure d'une couche de séparation mécanique.

6. Pansement médicamenteux selon l'une au moins des revendications précédentes, caractérisé en ce que des glycérides partiels à chaîne moyenne ou des mélanges de glycérides partiels sont introduits dans la masse de support.

7. Pansement médicamenteux selon l'une au moins des revendications précédentes, caractérisé en ce que le pansement est constitué par une feuille plastique autocollante sur une face, sur la face inférieure de laquelle se trouve un anneau de mousse à pores fermés, autocollant sur une face et le disque de masse de support assemblé à la feuille plastique se trouve dans la découpe de l'anneau en mousse.

8. Pansement médicamenteux selon l'une au moins des revendications précédentes, caractérisé en ce qu'on peut utiliser comme substances médicamenteuses par exemple celles destinées au traitement de l'hypertension sanguine et de l'angine de poitrine.

Fig.1

Fig.2

Fig.3

Fig.4

Fig.5